# EUROPÄISCHE PATENTSCHRIFT

(11) **EP 1 213 313 B1**
(45) Veröffentlichungstag und Bekanntmachung des Hinweises auf die Patenterteilung: **09.05.2007**
(21) Anmeldenummer: 02004371.7
(22) Anmeldetag: 02.12.1998
(51) Int. Cl.: C08G 59/18, C08L 63/00, A61K 6/087

(54) **Lagerstabile kationisch polymerisierende Zubereitungen mit verbessertem Härtungsverhalten**
Storage stable cationically polymerisable preparations with improved hardening characteristics
Préparations stables au stockage et polymérisables par voie cationique ayant des propriétés améliorées de durcissement

(30) Priorität: 02.12.1997 DE 19753461
(43) Veröffentlichungstag der Anmeldung: 12.06.2002
(62) Teilanmeldung aus: 98964480.2
(73) Patentinhaber: 3M ESPE AG, 82229 Seefeld (DE)
(72) Erfinder: Eckhardt, Gunther, Dr., 06231 Bad Duerrenberg (DE); Lechner, Günther, Dr., 82237 Wörthsee (DE); Wanek, Erich, Dr., 86916 Kaufering (DE); Somnitz, Ursula, 82362 Weilheim (DE)

(56) Entgegenhaltungen:
- DATABASE CHEMABS [Online] CHEMICAL ABSTRACTS SERVICE, COLUMBUS, OHIO, US; KOYAMA, TORU ET AL.: "Epoxy resin compositions with good balance of storage stability and curability" XP002214861 & IN 162 158 A (HITACHI) 9. April 1988 (1988-04-09)

## Beschreibung

Die Erfindung betrifft lagerstabile kationisch polymerisierende Zubereitungen, die ein verbessertes Härtungsverhalten besitzen. Die Zubereitungen basieren auf Verbindungen, die Epoxidgruppen und/oder N-Alkylaziridino-Gruppen und/oder Vinylether-Gruppen enthalten.

Es ist bekannt, daß die Polymerisation kationisch polymerisierbarer Verbindungen durch Substanzen mit sauren Eigenschaften ausgelöst werden kann (H.-G. Elias, "Makromoleküle", Hüthig u. Wepf Verlag, (1990)).

Für die Anwendung von kationisch polymerisierenden Zubereitungen ist es wichtig, daß die Polymerisation zum gewünschten Zeitpunkt beginnt und mit einem von der jeweiligen Anwendung abhängigen Härtungsverlauf beendet wird.

Für die Herstellung, Lagerung und die Anwendung kationisch polymerisierender Zubereitungen ist es notwendig, eine unerwünschte vorzeitige Polymerisation zu verhindern und den gewünschten Verlauf der Umsetzungsgrad- / Zeit-Kurve nach der Initiierung einzustellen.

Es ist bekannt, daß die kationische Polymerisation durch basische Substanzen verzögert oder verhindert wird. So ist es bekannt (DE-A-195 34 594), durch Zugabe von stickstoffhaltigen Verbindungen und insbesondere von Aminen, eine stabilisierende Wirkung zu erreichen. Die Amine besitzen meist eine ausreichende Löslichkeit in den zu stabilisierenden Zubereitungen und verzögern die Polymerisation. Die verzögernde Wirkung der Amine ist jedoch während der gesamten Aushärtungszeit vorhanden und führt meist zu einem verringertem Umsetzungsgrad der kationisch polymerisierbaren Gruppen, was in der Regel mit einer Verschlechterung der mechanischen Eigenschaften der ausgehärteten Massen verbunden ist.

Aus dem Abstract zu IN162158 (DATABASE CHEMABS CHEMICAL ABSTRACTS SERVICE, COLUMBUS, OHIO, US; KOYAMA, TORU ET AL.: "Epoxy resin compositions with good balance of storage stability and curability" XP002214861) sind Epoxidharzzusammensetzungen bekannt, die 0,0005-0,0010 % Alkali- und/oder Erdalkalimetallverbindungen enthalten, und eine verbesserte Lagerstabilität bei 40°C bzw. 160 und 170°C aufweisen.

Aufgabe der vorliegenden Erfindung ist es, kationische polymerisierende Zubereitungen zur Verfügung zu stellen, die einerseits lagerstabil sind und bei denen andererseits die für die Lagerstabilität verantwortlichen Stabilisatoren die Polymerisation nicht in unerwünschter Weise behindern.

Die Aufgabe wird gelöst durch kationisch polymerisierende Zubereitungen, die dadurch gekennzeichnet sind, daß sie 0,0005 bis 50 Gew.-% an löslichen und/oder feinteiligen organischen und/oder anorganischen Erdalkali- und/oder Alkalimetallverbindungen enthalten.

Überraschenderweise wurde festgestellt, daß sich die verzögernde Wirkung der löslichen und/oder feinteiligen organischen und/oder anorganischen Erdalkali- und/oder Alkalimetallverbindungen bei fortschreitender Polymerisation stark verringert. Diese verzögernde Wirkung zu Beginn der kationischen Polymerisation und deren Verringerung bei fortschreitender Polymerisation kann sowohl zur Verlängerung der Verarbeitungszeit nach erfolgter Initiierung als auch zur Erzeugung lagerstabiler kationisch polymerisierbarer Zubereitungen ohne den Nachteil eines verringerten Umsetzungsgrades genutzt werden. Die erfindungsgemäße Verwendung der erwähnten Erdalkaii- und/oder Alkaliverbindungen gestattet die Herstellung von lagerstabilen kationisch polymerisierenden Zubereitungen und darüber hinaus die Einstellung des Härtungsverlaufs und insbesondere der Verarbeitungszeit der initiierten Zubereitung bei Raumtemperatur sowie der zur Erreichung der Weiterverarbeitbarkeit der aushärtenden Masse notwendigen Zeit.

Die erfindungsgemäßen, kationisch polymerisierenden Zubereitungen basieren vorzugsweise auf Monomeren, die Epoxidgruppen und/oder N-Alkylaziridinogruppen und/oder Vinylethergruppen enthalten.

Als Epoxidgruppen-haltige Monomere kommen aromatische, aliphatische und cycloaliphatische Epoxyverbindungen in Betracht. Typische Vertreter dieser Monomeren sind die Glycidylether von Bisphenolen oder Novolaken sowie von aliphatischen Alkanolen, Alkandiolen oder Polyetherdiolen.

Die cycloaliphatische Epoxidgruppen enthaltenden Monomeren werden vorzugsweise ausgewählt aus der Gruppe (1) der Diepoxide cycloaliphatischer Ester der allgemeinen Struktur 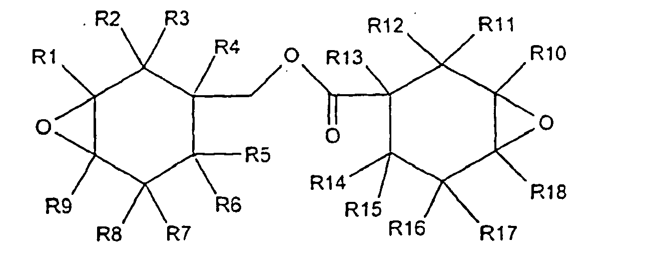 wobei die Substituenten R₁ bis R₁₈ gleich oder verschieden sein können und unabhängig voneinander H, Alkyl mit 1 bis 12 C-Atomen oder Aryl mit 6 bis 15 C-Atomen bedeuten, (2) der Produkte der Umsetzung von epoxidierten Cyclohexanderivaten des Alkohol- und Säuretyps mit aliphatischen Dicarbonsäuren oder Diolen sowie (3) der cycloaliphatisch substituierten Dioxyspiroalkane.

Besonders bevorzugt eingesetzte cycloaliphatische Diepoxidverbindungen sind 3,4-Epoxycyclohexylmethanol-3',4'-epoxycyclohexylcarboxylat und 3-(3',4'-Epoxycyclohexyl)-8,9-epoxy-2,4-dioxyspiro(5.5)-undecan.

Diese und andere erfindungsgemäß einsetzbare cycloaliphatische Diepoxide sind beispielsweise in der EP-B-0 119 425 beschrieben.

Die Herstellung und kationische Polymerisation von N-Alkylaziridinoverbindungen ist seit langem bekannt und wird von H. Bestian in Methoden der Organischen Chemie" (Houben Weyl) XII/ 1(1958) zusammenfassend beschrieben. Die DE-C-17 45 810 beschreibt die Synthese von Aziridinopolyethern und die Herstellung von Formkörpern auf der Basis der kationischen Polymerisation dieser Aziridinopolyether. Aziridinopolyether werden in dentalen Zubereitungen und insbesondere in Abformmaterialien eingesetzt.

Die kationische Polymerisierbarkeit von Vinylethern ist seit langer Zeit bekannt und wird heute zur Oberflächenveredelung, beispielsweise in Beschichtungsmassen mit sehr hoher Reaktivität, genutzt. Typische Vertreter von monomeren Vinylethern sind: Monovinylether aliphatischer verzweigter und unverzweigter Alkohole, wie n-Butylvinylether, Octadecylvinylether, Cyclohexylvinylether, tert.-Amylvinylether, Butandiolmonovinylether, Divinylether von Ethylenglykol und verschiedenen Oligoethylenglykolen, sowie Hexandiol und Trivinylether von Trimethylolpropan.

Die genannten Typen und Individuen der kationisch polymerisierbaren Monomeren sind sowohl allein als auch im Gemisch einsetzbar.

Allerdings sind bei der Auswahl sowohl die unterschiedliche Reaktivität als auch der Umstand zu beachten, daß die kationische Polymerisation an unterschiedlichen kationischen Zentren ablaufen kann.

Die erfindungsgemäßen Zubereitungen enthalten weiterhin die zur Polymerisationsauslösung geeigneten Verbindungen. Je nach der Anzahl der Komponenten, in die die Zubereitungen zur Erzielung ausreichender Lagerfähigkeit aufgeteilt werden müssen und in Abhängigkeit von den Eigenschaften der Monomeren kommen unterschiedliche Verbindungsklassen in Betracht.

So können unter erfindungsgemäßer Verwendung von löslichen und/oder feinteiligen organischen und/oder anorganischen Erdalkali- und/oder Alkaliverbindungen einkomponentige lagerstabile Zubereitungen hergestellt werden, die sowohl die beschriebenen einzelnen Monomeren oder Gemische einzelner Monomertypen und Individuen sowie Photoinitiatoren vom Typ der Oniumverbindungen und/oder der Metalloceniumverbindungen - jeweils mit einem komplexen Anion geringer Nucleophilie, enthalten.

Typische Vertreter der Oniumverbindungen, die bei Bestrahlung mit Licht einer Wellenlänge von 280 bis 400 nm zerfallen, sind Bisaryliodonium-Verbindungen und Trisarylsulfonium-Verbindungen.

Das Metallocenium-Kation kann in sehr unterschiedlicher Weise aufgebaut sein, wie es beispielsweise in der EP-A-0 542 716 dargestellt ist. Für den Einsatz in den erfindungsgemäßen Massen ist es zweckmäßig, solche Kationen auszuwählen, die bei Bestrahlung mit Licht einer Wellenlänge von 300 bis 550 nm unter Bildung von Lewis-Säuren oder Brönsted-Säuren zerfallen. Diese Bedingung kann durch MetalloceniumVerbindungen mit Eisen als Zentralatom in technisch nutzbarer Weise erfüllt werden.

Als Anionen können beispielsweise das Hexafluorophosphat- oder das Hexafluoroantimonat-Anion eingesetzt werden. Weiterhin kommen komplexe Borat-Anionen der allgemeinen Struktur

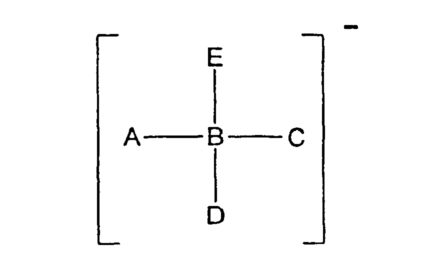

in Betracht, wobei die Substituenten A, E, C, D, gleich oder verschieden sein können und Aryl- oder Perfluoroaryl- bedeuten. Ein bevorzugt verwendetes Anion ist das Tetrakis(pentafluorophenyl)borat

Die als Photoinitiatoren verwendeten Verbindungen werden vorzugsweise in Konzentrationen von 0,1 bis 2 Gew.-%, besonders bevorzugt 0,4 bis 1,0 Gew.-%, der jeweiligen Zubereitungen eingesetzt.

Die erfindungsgemäß Erdalkali- und/oder Alkaliverbindungen enthaltenden einkomponentigen Zubereitungen zeichnen sich durch eine gute Lagerbeständigkeit aus, die durch Art und Konzentration der Erdalkali- und/oder Alkaliverbindungen auf Werte zwischen 12 und 60 Monaten bei 23° C eingestellt werden kann.

Falls technologisch erforderlich, kann die Dauer der Verzögerungsperiode nach der Belichtung der Zubereitungen am Beginn der Polymerisation durch Art und Konzentration der Erdalkali- und/oder der Alkaliverbindungen auf den gewünschten Wert eingestellt werden. Die erforderliche Aushärtungsgeschwindigkeit nach der Verzögerung ist durch eine ausreichende Photoinitiatorkonzentration und ggf. durch Anwendung mäßig erhöhter Temperaturen erreichbar. Somit lassen sich durch Zusatz der Erdalkali- und/oder Alkaliverbindungen einkomponentige, kationisch härtende Zubereitungen formulieren, die eine ausreichende Lagerstabilität besitzen und deren Härtungsverlauf auf die jeweilige Anwendung eingestellt werden kann.

Die kationisch polymerisierenden Zubereitungen können auch in zwei Teilzubereitungen aufgeteilt werden, wobei die sogenannte Katalysatorkomponente die polymerisationsauslösenden Spezies, ggf. in einem Verdünnungsmittel, und die sogenannte Basiskomponente die Monomeren enthält. Die erfindungsgemäß einzusetzenden Erdalkali- und/oder Alkaliverbindungen können sowohl der Katalysatorkomponente als auch der Basiskomponente zugegeben werden. Die Zugabe zur Basiskomponente ist die bevorzugte Ausführungsform.

Prinzipiell können bei einer zweikomponentigen Ausführungsform in der Katalysatorkomponente Brönsted- und bzw. oder Lewis-Säuren eingesetzt werden. Geeignete Säuren sind beispielsweise Hexafluoroantimonsäure, Hexafluorophosphorsäure, Tetrafluoroborsäure, p-Toluolsulfonsäure, Benzolsulfonsäure und Alkansulfonsäuren.

Es können aber auch Systeme eingesetzt werden, deren Einzelkomponenten auf die Teilzubereitungen aufgeteilt werden und die beim Vermischen der Teilzubereitungen die eigentlichen polymerisationsauslösenden Spezies, wie beispielsweise die Säuren, erzeugen. So können beispielsweise in der Katalysatorkomponente nichtphotosensitive Sulfoniumverbindungen wie sie in DE-A-25 15 593 beschrieben sind, verwendet werden, die nach Kontakt mit der Aziridinoverbindung der Basiskomponente eine polymerisationsauslösende Spezies vom Typ eines Aziridiniumsalzes bilden.

Weiterhin kann die Katalysatorkomponente Aziridiniumsalze enthalten, die zur Auslösung der kationischen Polymerisation der entsprechend ausgewählten Monomeren geeignet sind. Geeignete Aziridiniumsalze sind durch Umsetzung der Aziridinoverbindungen mit den vorgenannten Säuren zugänglich.

Die kationisch härtenden, einkomponentigen oder zweikomponentigen Zubereitungen enthalten erfindungsgemäß 0,0005 bis 50 Gew.-% an Erdalkali- und/oder Alkaliverbindungen.

Die Erdalkali- und Alkalimetallverbindungen können sowohl in gelöster als auch in feinteiliger, fester Form in die Zubereitungen eingebracht werden. Es ist auch möglich, mit Erdalkali- und/oder Alkalimetallverbindungen dotierte oder Erdalkali- und/oder Alkaliionen enthaltende anorganische Füllstoffe wie Silikate, Quarz, Diatomeenerde oder feinteilige organische Polymere, die Alkaliverbindungen in adsorbierter Form oder die Alkalimetallionen in gebundener Form enthalten, erfindungsgemäß zu verwenden.

Vorzugsweise werden lösliche organische und/oder anorganische Erdalkali- und/oder Alkalimetallverbindungen mit Molmassen unter 1000 g/Mol in einer Menge von 0,01 bis 20 Gew.-% eingesetzt. Bevorzugt ist weiterhin der Einsatz von hochpolymeren Verbindungen in einer Menge von 1 bis 50 Gew.-% mit einem Erdalkali- und/oder Alkaligehalt von 0,01 bis 10 Gew.-%.

Der Einsatz von Alkalimetallalkylverbindungen wie beispielsweise Butyllithium ist möglich. Bevorzugt werden jedoch Erdalkali- und/oder Alkalimetallalkoholate eingesetzt, wie sie durch Umsetzung ausgesuchter, bevorzugt primärer, ein- oder mehrwertiger Alkohole zu den entsprechenden Alkylaten zugänglich sind.

Typische Vertreter dieser Verbindungsklasse sind: Lithium-2-ethylhexylalkoholat, Lithiumlaurylalkoholat, Natriumalkoholat des Polytetrahydrofurandiols mit einer Molmasse von 350 g/Mol, Lithiumalkoholat eines Mischpolyetherglykols aus Tetrahydrofuran- und Ethylenoxid-Einheiten mit einer Molmasse von 3000 bis 8000 und vorzugsweise von 6000 g/Mol.

Eine besonders bevorzugte Verbindungsklasse der Erdalkali- und Alkaliverbindungen sind die Erdalkali- und/oder Alkalisalze von gesättigten oder ungesättigten Carbonsäuren, wobei die Carbonsäuren ein- oder mehrwertig sowie aliphatisch, olefinisch oder aromatisch sein können. Typische Vertreter dieser Verbindungsklasse sind: Calciumstearat, Calciumoleat, Strontiumoleat, Lithium-2-ethylhexanolat, Natriumpalmitat, Natriumstearat, Kaliumerucat, Natriumricinolat, Lithiumoleat, Lithiumdodecylbenzoat. Dieser Verbindungsklasse gehören auch die Erdalkali- und/oder die Alkalimetallcarboxylate gesättigter oder ungesättigter Carbonsäuren an, die aus oligomeren ein- und bevorzugt mehrwertigen Säuren und den entsprechenden Hydroxiden, Alkylen oder Alkoxiden zugänglich sind. Alle diese Verbindungen werden in Mengen von vorzugsweise 0,01 bis 20 Gew.-% eingesetzt.

Solche oligomeren Säuren können beispielsweise carboxylfunktionalisierte Polyether, Polyester oder Acrylnitril-Butadien-Copolymerisate mit Molmassen von 500 bis 5000 g/Mol sein. Vorteilhaft einsetzbare, weil in der Basispaste gut lösliche Alkalicarboxylate sind aus Polyether- oder Polyesterpolyolen durch vollständige oder partielle Umsetzung der OH-Gruppen mit dem Anhydrid einer zweiwertigen Säure und anschließende Neutralisation mit Alkalihydroxiden, Alkalialkylen oder Alkalialkoxiden zugänglich.

Ein typischer Vertreter dieser Verbindungsklasse ist das Umsetzungsprodukt eines Caprolactontriols mit der Molmasse 540 g/Mol mit Maleinsäureanhydrid im Verhältnis OH-Gruppen: Anhydridgruppen von 1 : 0,4, das nachfolgend mit Lithiumhydroxid oder Lithiumalkoxid in das Lithiumcarboxylat umgesetzt wird.

Bevorzugt werden weiterhin 0,01 bis 20 Gew.-% an Erdalkali- und/oder Erdalkalisalzen der Umsetzungsprodukte von cyclischen Anhydriden mit ein- und/oder mehrwertigen Alkoholen eingesetzt, wobei Maleinsäureanhydrid als cyclisches Anhydrid und als mehrwertige Alkohole Triole mit Molmassen über 500 g/Mol für die Umsetzung bevorzugt werden. Vorzugsweise erfolgt dabei die Umsetzung der OH-Gruppen der Triole mit dem Maleinsäureanhydrid nur partiell.

Als Alkalimetallverbindungen werden diejenigen des Kaliums, Natriums und/oder Lithiums, insbesondere des Lithiums bevorzugt. Als Erdalkalimetallverbindungen werden diejenigen des Calciums und Strontiums bevorzugt.

Die an einen Feststoff adsorbierten Erdalkali- und bzw. oder Alkaliverbindungen bzw. die Füllstoffe, die Erdalkali- und bzw. oder Alkaliionen enthalten, werden bevorzugt gegen Ende des Mischvorgangs portionsweise in die Basiskomponente eingebracht. Die Zugabe mancher Alkoholate oder Carboxylate erfolgt zweckmäßigerweise als vorgefertigte Paste.

Beispielsweise können Calciumstearat, Calciumoleat, Natriumpalmitat, Lithiumricinolat, Lithiumerucat oder Lithiumoleat in Polyetherglykolen ggf. unter Zusatz von Wasser angeknetet und durch Dissolver oder auf dem Walzenstuhl in eine pastenförmige Konsistenz gebracht werden.

Die Zugabe dieser Verzögererpasten kann zu jedem Zeitpunkt der Basiskomponenten-Herstellung erfolgen, wird aber vorteilhafterweise gegen Ende der Knetung realisiert.

Die Erfindung wird anhand der nachfolgenden Beispiele näher erläutert.

Die erfindungsgemäßen Zubereitungen können zum Verkleben, Abdichten, Vergießen und Beschichten von Substraten, weiterhin in dentalmedizinischen und dentaltechnischen Präparaten, sowie zur Abformung von Gegenständen und insbesondere zur dentalen Abformung verwendet werden.

### Beispiele

### Beispiele 1 bis 4

Die Beispiele 1 bis 4 betreffen einkomponentige Zubereitungen, während zweikomponentige Zubereitungen in den Beispielen 5 bis 15 beschrieben sind.

Die Beurteilung der Lagerfähigkeit der monomerhaltigen Zubereitungen erfolgte durch Viskositätsmessungen und Kontrolle der Aushärtung. Es wurde die Lagerfähigkeit bei 23°C bestimmt und in Monaten angegeben. Innerhalb der angegeben Zeit stieg die Viskosität der monomerhaltigen Zubereitung, verglichen mit dem Ausgangswert, um weniger als 15 % an, und die Aushärtungsgeschwindigkeit sowie die Eigenschaften des ausgehärteten Materials änderten sich nicht.

Der Polymerisationsbeginn" wurde als der Zeitpunkt bestimmt, bei dem eine belichtete einkomponentige Zubereitung oder eine gemischte zweikomponentige Zubereitung deutliche Veränderungen, wie Hautbildung, Fädenziehen und stark verringerte Fließfähigkeit, zeigt. Die Zeit bis zum "Polymerisationsbeginn" wird als "Verarbeitungszeit" angesehen.

Als "Polymerisationsende" wird der Zeitpunkt definiert, bei der nach Belichtungsbeginn die belichtete einkomponentige Zubereitung bzw. nach Mischbeginn die gemischte zweikomponentige Zubereitung sich soweit verfestigt hat, daß der entstandene Festkörper die fertigungstechnisch üblichen mechanischen Beanspruchungen übersteht und die "Weiterverarbeitbarkeit" gegeben ist. Üblicherweise ist die innere Festigkeit des Festkörpers zu diesem Zeitpunkt auf etwa 50 bis 80 % des Endwertes angestiegen. Die Endwerte der mechanischen Eigenschaften werden meist erst nach mehreren Stunden erreicht.

Zur Aushärtung der einkomponentigen Zubereitungen wurden diese in einer kreisförmigen Form aus Teflon mit 2 mm Prüfkörperhöhe und einem Durchmesser von 20 mm mit einer Lampe, die Licht im Wellenlängenbereich von 280 bis 550 mm emittiert, wobei die Strahlungsintensität auf der Ebene der Prüfkörperoberfläche einen Wert von 45 mW/cm² besitzt, belichtet.

Mit einem Spatel wurde durch wiederholtes Eintauchen in die belichtete Zubereitung der Belichtungs-Zeitpunkt bestimmt, bei dem die beschriebenen Anzeichen des "Polymerisationsbeginns" auftraten. Zur Ermittlung des "Polymerisationsendes" wurde die Belichtung noch weitere 30 Sekunden über den "Polymerisationsbeginn" hinaus fortgesetzt.

Fünf Minuten nach Belichtungsbeginn wurde der Prüfkörper entformt und seine Festigkeit beurteilt.

Für genauere Messungen wurden anhand des beschriebenen Belichtungsmodus Prüfkleinstäbe mit den Abmessungen der Prüfstrecke 10 x 2 x 2 mm hergestellt. Die notwendige Mindestfestigkeit ist stark abhängig vom jeweiligen Anwendungsfall.

Die Untersuchung der Prüfkörper gemäß den Beispielen 1 bis 4 ergab am "Polymerisationsende" Zugfestigkeitswerte, die größer als 2 N/mm² waren.

**Tabelle 1:**

| **Zusammensetzung der erfindungsgemäßen einkomponentigen Zubereitungen** | | | | | | |
|---|---|---|---|---|---|---|
| **Beispiel** | **Monomere** | **Gew.-%** | **Erfindungsgemäße Stabilisatoren/ Verzögerer** | **Gew.-%** | **Sonstige Bestandteile** | **Gew. -%** |
| 1 | * 3,4-Epoxycyclohexyl-methyl-3',4'-epoxyhexyl-carboxylat | 57,43 | * Umsetzungsprodukt von Polycaprolactontriol (Molmasse 540 g / mol) mit Maleinsäureanhydrid im Molverhältnis 1 : 0,4 und nachfolgende Neutralisation mit Lithiummethylat | 0,11 | * Polycaprolactontriol (M = 540 g/mol) | 38,9 |
| | | | | | * 8-Hydroxychinolin | 0,01 |
| | | | | | * Ferroceniumhexafluoroantimonat | 0,70 |
| | | | | | * Cumolhydroperoxid (80%-ig) | 2,85 |
| 2 | * Trisaziridino-Polyether mit einer Iminoäquivalent-masse von 2050, enthaltend Ethylenoxid- und Tetrahydrofuran-Einheiten im Verhältnis 1 : 3,4 | 60,15 | * Umsetzungsprodukt eines Mischpolyelherdiols mit der Molmasse 5900 g/mol, enthaltend Ethylenoxid- und Tetrahydrofuran -Einheiten im Verhältnis 1 : 3,4 mit Butyllithium | 5,7 | * Dibenzyltoluol | 11,70 |
| | | | | | * Ferroceniumtosylat | 1,45 |
| | | | | | * Cumolhydroperoxid | 0,50 |
| | | | | | * Quarzgutmeht, silanisiert | 20,50 |
| 3 | * 3,4-Epoxycyclohexyl-methyl-3',4'-epoxyhexyl-carboxylat | 4,7 | * Umsetzungsprodukt wie bei Beispiel 1, jedoch neutralisiert mit Natriummethylat | 0,07 | * Mischpolyetherdiol mit einer Molmasse von 5900 g/mol, enthaltend Ethylenoxid- und Tetrahydrofuran-Einheiten | 10,1 |
| | * Divinylether des 1,4-Cyclohexandimethanols | 84,33 | | | im Verhältnis 1 : 3,4 | |
| | | | | | * Trisarylsulfoniumhexafluoroanlimonal | 0,8 |
| 4 | * 3,4-Epoxycyclohexyl-methyl-3',4'-epoxyhexyl-carboxylat | 31,7 | * Umsetzungsprodukt von Polyethylenglykoldicarbonsäure mit einer Molmasse von 600 mit Lithium-hydroxid | 0,09 | * Polycarbonatdiol mit einer Molmasse von 550 g/mol | 5,86 |
| | | | | | * Trisarylsulfoniumhexafluoroanlimonat | 1,25 |
| | * Epoxidiertes Polybutadien mit einer Epoxyäquivalentmasse von 450 g/mol | 20,1 | | | * Quarzgutmehl, silanisiert | 41,0 |

**Tabelle 2**

| **Prüfergebnisse der Zubereitungen gemäß den Beispielen 1 bis 4 (siehe Tabelle 1)** | | | |
|---|---|---|---|
| **Zubereitung gemäß Beispiel** | **Lagerbeständigkeit bei 23° C / Monate** | **"Polymerisationsbeginn" Sekunden** | **"Polymerisationsende" Sekunden** |
| **1** | größer 24 | 18 | 20 |
| **2** | größer 24 | 40 | 80 |
| **3** | 18 | 13 | 16 |
| **4** | 18 | 11 | 13 |

### Vergleichsbeispiele 1 bis 4

Die Herstellung der Zubereitungen gemäß den Beispielen 1 bis 4 wurde wiederholt mit dem Unterschied, daß jeweils die erfindungsgemäßen Stabilisatoren / Verzögerer weggelassen wurden.

### Vergleichsbeispiel 5

Zur Herstellung der Zubereitung gemäß Vergleichsbeispiel 5 wurde in der Rezeptur des Beispieles 1 der erfindungsgemäße Stabilisator / Verzögerer ersetzt durch Butoxyethyl-4-dimethyl-amino-benzoat in gleicher Konzentration.

Die so erzeugten Zubereitungen gemäß den Vergleichsbeispielen 1 bis 5 wurden unter den gleichen Bedingungen wie bei den Beispielen 1 bis 4 ausgehärtet.

Die Prüfergebnisse der Vergleichsbeispiele 1 bis 5 sind in Tabelle 3 zusammengestellt.

**Tabelle 3**

| **Prüfergebnisse der Zubereitungen gemäß den Vergleichsbeispielen 1 bis 5** | | | |
|---|---|---|---|
| **Zubereitung gemäß Vergleichsbeispiel** | **Lagerbeständigkeit bei 23° C / Monate** | **"Polymerisationsbeginn" Sekunden** | **"Polymerisationsende" Sekunden** |
| **1** | 8 | 12 | 15 |
| **2** | 12 | 10 | 60 |
| **3** | 6 | 10 | 12 |
| **4** | 3 | 8 | 12 |
| **5** | 18 | 16 | 36 |

Der Vergleich der Ergebnisse der Beispiele 1 bis 4 mit denen der Vergleichsbeispiele 1 bis 5 zeigt, daß sowohl mit den erfindungsgemäß eingesetzten Verbindungen als auch mit einem Amin (Vergleichsbeispiel 5) eine Verbesserung der Lagerbeständigkeit erreichbar ist. Die erfindungsgemäß eingesetzten Verbindungen bewirken eine verarbeitungstechnisch relevante Verlängerung der Verarbeitungszeit bei nur geringfügig verlängertem "Polymerisationsende", während durch den Einsatz des Amins das "Polymerisationsende" stark verzögert wird.

**Tabelle 4**

| **Zusammensetzung der erfindungsgemäßen zweikomponentigen Zubereitungen, Teil : Katalysatorkomponenten (Gew.-%)** | | | | |
|---|---|---|---|---|
| **Katalysator-komponente** | **Polymerisationsauslösende Spezies oder Teil des 2K-Initiierungssystems** | **Gew.-%** | **Sonstige Bestandteile** | **Gew.-%** |
| K1 | * Hexafluoroantimonsäure | 0,3 | * Polycaprolactontriol mit einer Molmasse von 540 g/mol | 98,8 |
| | | | * Farbpaste, rot | 0,2 |
| | | | * Hydrophile, pyrogene Kieselsäure | 0,7 |
| K2 | * Bis-aziridiniumhexafluoroantimonat eines Mischpolyetherdiols mit einer Molmasse von 5900 g / mol, enthaltend Ethylenoxid- und | 64,0 | * Polypropylenglykol mit einer Molmasse von 3000 | 35,7 |
| | Tetrahydrofuraneinheiten im Verhältnis 1 : 3,4 | | * Farbpaste, rot | 0,3 |
| | | | | |
| K3 | * Hexafluoroantimonsäure | 0,5 | * Mischpolyetherdiol mit einer Molmasse von 5900 g/mol, enthaltend Ethylenoxid- und Tetrahydrofuran-Einheiten im Verhältnis 1 : 3,4 | 99,2 |
| | | | * Farbpaste, rot | 0,3 |
| K4 | * Toluolsulfonsäure-Monohydrat | 10,0 | * Polypropylenglykol mit einer Molmasse von 8000 g/mol | 20,0 |
| | | | * Poly(ethylen, propylen-)glykol mit einer Molmasse von 3000 g/mol | 62,5 |
| | | | * Hydrophile, pyrogene Kieselsäure | 7,0 |
| | | | * Farbpaste, rot | 0,5 |
| K5 | * p-Toluolsulfonsäure-Monohydrat | 9,0 | * Poly(ethylen, propylen-)glykol mit einer Molmasse von 3000 g / mol | 82,5 |
| | | | * Hydrophile, pyrogene Kieselsäure | 8,0 |
| | | | * Farbpaste, rot | 0,5 |

**Tabelle 5**

| **Zusammensetzung der erfindungsgemäßen, zweikomponentlgen Zubereitungen; Teil: Basiskomponenten und Ihre Lagerbeständigkeit (Gew.-%)** | | | | | | | |
|---|---|---|---|---|---|---|---|
| **Basis-komponente** | **Monomere** | **Gew.-%** | **Erfindungsgemäße Stabilisatoren/ Verzögerer** | **Gew.-%** | **Sonstige** | **Ge w.-%** | **Lagerbeständigkeit bei 23°C/Monate** |
| B1 | * 3,4-Epoxycyclohexylmethyl-3',4'.epoxycyclohexylcarboxylat | 93,38 | * Umsetzungsprodukt eines Polycaprolactontriols mit einer Molmasse von 540 g/mol mil Maleinsäureanhydrid im Molverhältnis 1 : 0,35 und anschließende Neutralisation mit Lithiummethylat | 0,12 | * feindisperse Kieselsäure, silanisiert | 2,1 | > 18 |
| | * Hydroxybulylvinylether | 3,7 | | | * Farbpaste, blau | 0,7 | |
| B2 | * 3,4-Epoxycyclohexylmethyl-3',4'.epoxycyclohexylcarboxylat | 75,55 | * Umsetzungsprodukt eines Polycaprolactontriols mit einer Molmasse von 540 g/mol mit Maleinsäure-anhydrid im Molverhältnis 1 : 0,35 und anschließende Neutralisation mit Lithiummethylat | 0,05 | * feindisperse Kieselsäure, silanisiert | 1,7 | > 18 |
| | * Glycidyloxypropyltrimethoxysilan | 1,0 | | | * Quarzgutmehl, silanisiert | 21,0 | |
| | | | * Calciumstearat | 0,10 | * Farbpaste, blau | 0,6 | |
| B3 | * Bis-N-alkylaziridinoderivat eines Mischpolyethers mit einer Molmasse von 6100, enthaltend Ethylenoxid- und Tetrahydrofuran-Einheiten im Verhältnis 1 : 3,6 | 58,6 | * Lilhiumoleat | 2,0 | * Mischpolyelherglykol mit einer Molmasse von 5900 | 3,5 | > 24 |
| | | | | | * hydrierter Rindertalg | 11,2 | |
| | | | | | * Kieselgur | 14,3 | |
| | | | | | * Dibenzyltoluol | 10,4 | |
| | | | | | * Farbpaste, blau | 0,7 | |
| B4 | * Bis-N-alkylaziridinoderivat eines Mischpolyethers mit einer Molmasse von 6100, enthaltend Ethylenoxid- und Tetrahydrofuran-Einheiten Im Verhältnis 1 : 3.6 | 57,3 | * Calciumstearat | 1,5 | * Poly(ethylen, propylen-)glykol | 8,3 | > 24 |
| | | | * Umsetzungsprodukt eines Mischpolyetherpolyols mit Butyllithium; Lithiumäquivalent-masse : 2100 g/mol | 5,0 | * hydrierter Rindertalg | 11,7 | |
| | | | | | * Kieselgur | 14,3 | |
| | | | | | * Dibenzylloluol | 1,2 | |
| | | | | | * Farbpaste, blau | 0,7 | |
| B5 | * 3,4-Epoxycyclohexylmethyl 3',4'.epoxycyclohexylcarboxylat | 68,7 | * Glaspulver, mit Salzsäure behandelt, mit Wasser gewaschen, In 0,5%-Iger alkoholischer Lösung von Lilhlumhydroxid 24 Stunden gelagert, getrocknet | 22,1 | * feindisperse Kieselsäure, silanisiert | 3,2 | >12 |
| | * Bis-N-alkylaziridinoderivat eines Mischpolyethers mit einer Molmasse von 6100, enthaltend Ethylenoxid- und Tetrahydrofuran-Einheiten ImVerhältnis 1 : 3,6 | 5,3 | | | * Farbpaste, blau | 0,7 | |

**Tabelle 6**

| **Zusammensetzung der erfindungsgemäßen zweikomponentigen Zubereitungen und Prüfergebnisse** | | | | | |
|---|---|---|---|---|---|
| **Beispiel** | **Katalysator-komponente (s. Tabelle 4)** | **Basis-komponente (s. Tabelle 5)** | **Mischungsverhältnis (nach Gewicht)** | **"Polymerisationsbeginn" Sekunden** | **"Polymerisationsende" Sekunden** |
| 5 | K1 | B1 | 1:1,2 | 20 | 600 |
| 6 | K1 | B2 | 1:1,2 | 15 | 550 |
| 7 | K2 | B3 | 1:5 | 45 | 180 |
| 8 | K2 | B4 | 1:5 | 60 | 210 |
| 9 | K3 | B5 | 1:2 | 125 | 430 |
| 10 | K4 | B3 | 1:4,5 | 140 | 350 |
| 11 | K4 | B4 | 1:5 | 150 | 390 |
| 12 | K4 | B4 | 1:4 | 120 | 360 |
| 13 | K5 | B3 | 1:4 | 110 | 350 |
| 14 | K5 | B3 | 1:5 | 130 | 370 |
| 15 | K5 | B4 | 1:4,5 | 125 | 340 |

### Beispiele 5 bis 15

Die zweikomponentigen Zubereitungen gemäß den Beispielen 5 bis 15 wurden durch Mischen der Katalysatorkomponenten (Tabelle 4) und der Basiskomponenten (Tabelle 5) entsprechend dem in Tabelle 6 genannten Mischungsverhältnis hergestellt und die Zeit bis zum "Polymerisationsbeginn" und bis zum "Polymerisationsende" in der beschriebenen Weise ermittelt, wobei die Mischzeit bei den Beispielen 5 und 6 10 Sekunden und bei den Beispielen 7 bis 15 30 Sekunden betrug. Die Ergebnisse der Bestimmung der Kennzahlen des Aushärteverlaufs sind in Tabelle 6 dargestellt. Die Lagerbeständigkeit der Basiskomponenten zur Erzeugung der zweikomponentigen Zubereitungen gemäß den Beispielen 5 bis 15 ist in Tabelle 5 enthalten.

### Vergleichsbeispiele 6 bis 16

Zur Herstellung der zweikomponentigen Zubereitungen gemäß den Vergleichsbeispielen 6 bis 16 wurden die Katalysatorkomponenten entsprechend Tabelle 4 in den in Tabelle 6 angegebenen Mischungsverhältnisse mit den Basiskomponenten gemäß Tabelle 5 mit dem Unterschied gemischt, daß für die Zubereitungen der Vergleichsbeispiele die erfindungsgemäßen Stabilisatoren in den Basispasten weggelassen wurden. Die Lagerbeständigkeit der Basiskomponenten ohne die erfindungsgemäßen Stabilisatoren betrug bei 23° C 4 bis 10 Monate.
Eine Zusammenstellung der Kennzahlen für den Aushärteverlauf der Zubereitungen gemäß den Vergleichsbeispielen 6 bis 16 enthält Tabelle 7.

**Tabelle 7**

| **Vergleichsbeispiele 6 bis 16** | | | | | |
|---|---|---|---|---|---|
| **Zusammensetzung der zweikomponentigen Zubereitungen und Zusammenstellung der Prüfergebnisse** | | | | | |
| **Vergleichsbeispiel** | **Katalysatorkomponente (s. Tabelle 4)** | **Basiskomponente a) (s. Tabelle 5)** | **Mischungsverhältnis (nach Gewicht)** | **"Polymorisationsbeginn" Sekunden** | **"Polymerisationsende" Sekunden** |
| 6 | K1 | B1 | 1:1,2 | < 10 (bereits während des Anmischens) | 400 |
| 7 | K1 | B2 | 1:1,2 | < 10 (bereits während des Anmischens) | 300 |
| 8 | K2 | B3 | 1:5 | 25 | 150 |
| 9 | K2 | B4 | 1:5 | 40 | 170 |
| 10 | K3 | B5 | 1:2 | 30 | 125 |
| 11 | K4 | B3 | 1:4,5 | 48 | 245 |
| 12 | K4 | B4 | 1:5 | 53 | 255 |
| 13 | K4 | B4 | 1:4 | 45 | 240 |
| 14 | K5 | B3 | 1:4 | 42 | 205 |
| 15 | K5 | B3 | 1:5 | 48 | 220 |
| 16 | K5 | B4 | 1:4,5 | 52 | 260 |

| | | | | | |
|---|---|---|---|---|---|
| a) jeweils ohne den erfindungsgemäßen Stabilisator (Verzögerer) | | | | | |

### Vergleichsbeispiel 17

Die Katalysatorkomponente K5 (s. Tabelle 4) wurde im Gewichtsverhältnis 1 : 5 mit einer Basiskomponente, enthaltend:
57,4 Gew.-% Bis-N-alkylaziridinopolyether gemäß B4
0,5 Gew.-% 1-Laurylimidazol
42,1 Gew.-% Sonstige Bestandteile in dem Verhältnis, wie bei der Basiskomponente B4 angegeben,
gemischt.

Es wurde ein "Polymerisationsbeginn" von 140 Sekunden und ein "Polymerisationsende" von 450 Sekunden ermittelt.

Die Lagerbeständigkeit der Basiskomponente betrug 24 Monate.

Der Vergleich der Ergebnisse der für die Beispiele 5 bis 15 eingesetzten Basiskomponenten (Tabelle 5), mit denen der Vergleichsbeispiele 6 bis 16 zeigt, daß sowohl mit den erfindungsgemäß verwendeten Verbindungen als auch mit einem Amin (s. Vergleichsbeispiel 17) die Lagerbeständigkeit der Basiskomponenten verbessert wird.

Die erfindungsgemäß eingesetzten Verbindungen bewirken eine für die Verarbeitung sehr wichtige Verlängerung der Zeit bis zum "Polymerisationsbeginn" bei einem tolerierbaren, verlängertem "Polymerisationsende", während durch den Einsatz des Amins (Vergleichsbeispiel 17) das "Polymerisationsende" wesentlich stärker verzögert wird.

## Patentansprüche

1. Verwendung einer kationisch polymerisierenden Zubereitung auf der Basis von Epoxidgruppen-haltigen Monomeren und zur Polymerisationsauslösung geeigneten Verbindungen, die **dadurch gekennzeichnet ist, daß** sie zur Einstellung des Härtungsverhaltens und zur Verbesserung der Lagerstabilität 0,0005 bis 50 Gew.-% an löslichen und/oder feinteiligen Erdalkali- und/oder Alkalisalzen gesättigter oder ungesättigter Carbonsäuren oder Erdalkali- und/oder Alkalisalzen der Umsetzungsprodukte von cyclischen Anhydriden mit ein- und/oder mehrwertigen Alkoholen enthält, oder 1 bis 50 Gew.-% an löslichen hochpolymeren Verbindungen enthält, die einen Erdalkali- und/oder Alkaligehalt von 0,01 bis 10 Gew.-% besitzen, in dentalmedizinischen und dentaltechnischen Präparaten sowie zur dentalen Abformung.

2. Verwendung nach Anspruch 1, **dadurch gekennzeichnet, daß** Maleinsäureanhydrid als cyclisches Anhydrid für die Umsetzung verwendet wird.

3. Verwendung nach den Ansprüchen 1 und 2, **dadurch gekennzeichnet, daß** als mehrwertige Alkohole Triole mit Molmassen über 500 g/Mol für die Umsetezung verwendet werden.

4. Verwendung nach Anspruch 3, **dadurch gekennzeichnet, daß** die Umsetzung der OH-Gruppen der Triole mit Maleinsäureanhydrid nur partiell erfolgt.

5. Verwendung nach den Ansprüchen 1 bis 4, **dadurch gekennzeichnet, daß** die Zubereitung als Alkalimetallverbindungen solche des Kaliums, Natriums und/oder Lithiums, vorzugsweise des Lithiums, und/oder als Erdalkaliverbindungen solche des Calciums und/oder des Strontiums enthält.

6. Verwendung nach den Ansprüchen 1 bis 5, **dadurch gekennzeichnet, daß** die Zubereitung eine wirksame Menge eines oder mehrerer Photoinitiatoren enthält.

7. Verwendung nach den Ansprüchen 1 bis 5, **dadurch gekennzeichnet, daß** die Zubereitung aus einer Basiskomponente und getrennt hiervon aus einer Katalysatorkomponente besteht, wobei die Basiskomponente das oder die Monomeren und die Katalysatorkomponente die die Polymerisation auslösenden Species, ggfs. in einem Verdünnungsmittel, enthalten, und wobei die Erdalkali- und/oder Alkaliverbindungen in der Basis- und/oder Katalysatorkomponente, vorzugsweise in der Basiskomponente vorliegen.

## Claims

1. Use of a cationically polymerising preparation based on monomers containing epoxide groups and on compounds suitable for initiating polymerisation, which is **characterised in that**, in order to adjust the curing behaviour and to improve the storage stability, it contains 0.0005 to 50 wt. % of soluble and/or fine-particle alkaline earth salts and/or alkali salts of saturated or unsaturated carboxylic acids, or alkaline earth salts and/or alkali salts of the reaction products of cyclic anhydrides with mono- and/or polyhydric alcohols, or contains 1 to 50 wt. % of soluble high-polymeric compounds which possess an alkaline earth and/or alkali content of 0.01 to 10 wt. %, in products in dental medicine and dentistry and also for dental impression.

2. Use according to Claim 1, **characterised in that** maleic anhydride is used as cyclic anhydride for the reaction.

3. Use according to Claims 1 and 2, **characterised in that** triols having molar masses above 500 g/mol are used as polyhydric alcohols for the reaction.

4. Use according to Claim 3, **characterised in that** the reaction of the OH groups of the triols with maleic anhydride takes place only partially.

5. Use according to Claims 1 to 4, **characterised in that** the preparation comprises as alkali metal compounds those of potassium, sodium and/or lithium, preferably of lithium, and/or as alkaline earth compounds those of calcium and/or of strontium.

6. Use according to Claims 1 to 5, **characterised in that** the preparation comprises an effective amount of one or more photoinitiators.

7. Use according to Claims 1 to 5, **characterised in that** the preparation is composed of a base component and, separately therefrom, of a catalyst component, the base component comprising the monomer or monomers and the catalyst component comprising the species initiating polymerisation, optionally in a diluent, and the alkaline earth and/or alkali compounds being present in the base component and/or catalyst component, preferably in the base component.

## Revendications

1. Utilisation, dans des préparations de médecine dentaire et de technique dentaire, ainsi que pour la prise d'empreintes dentaires, d'une préparation polymérisable par voie cationique, à base de monomères contenant des groupes époxy et des composés convenant à l'amorçage de la polymérisation, qui est **caractérisée en ce que** la préparation contient, pour ajuster le comportement en durcissement et pour améliorer la stabilité au stockage, de 0,0005 à 50 % en poids de sels de métaux alcalins et/ou alcalino-terreux, solubles et/ou finement divisés, d'acides carboxyliques saturés ou insaturés, ou des sels de métaux alcalins et/ou alcalino-terreux des produits de la réaction d'anhydrides cycliques avec des mono- et/ou polyalcools, ou de 1 à 50 % en poids de composés hauts polymères solubles, qui ont une teneur en métaux alcalins et/ou alcalino-terreux de 0,01 à 10 % en poids.

2. Utilisation selon la revendication 1, **caractérisée en ce qu'**on utilise de l'anhydride maléique en tant qu'anhydride cyclique pour la réaction.

3. Utilisation selon les revendications 1 et 2, **caractérisée en ce qu'**on utilise pour la réaction, en tant que polyalcools, des triols ayant une masse moléculaire supérieure à 500 g/mol.

4. Utilisation selon la revendication 3, **caractérisée en ce que** la réaction des groupes OH des triols avec l'anhydride maléique ne s'effectue que d'une manière partielle.

5. Utilisation selon les revendications 1 à 4, **caractérisée en ce que** la préparation contient, en tant que composés de métaux alcalins, ceux du potassium, du sodium et/ou du lithium, de préférence du lithium, et, en tant que composés de métaux alcalino-terreux, ceux du calcium et/ou du strontium.

6. Utilisation selon les revendications 1 à 5, **caractérisée en ce que** la préparation contient une quantité efficace d'un ou plusieurs photoamorceurs.

7. Utilisation selon les revendications 1 à 5, **caractérisée en ce que** la préparation est constituée d'un composant de base et, séparément de ce dernier, d'un composant catalyseur, le composant de base contenant le ou les monomères, et le composant catalyseur contenant l'espèce amorçant la polymérisation, éventuellement dans un diluant, et les composés de métaux alcalins et/ou alcalino-terreux étant présents dans le composant de base et/ou dans le composant catalyseur, de préférence dans le composant de base.
